# EUROPÄISCHE PATENTANMELDUNG

(11) **EP 4 302 732 A1**
(43) Veröffentlichungstag der Anmeldung: **10.01.2024**
(21) Anmeldenummer: 23183081.1
(22) Anmeldetag: 03.07.2023
(51) Int. Cl.: A61F 5/01

(54) **ORTHESE**

(30) Priorität: 04.07.2022 DE 102022116634
(71) Anmelder: Reha-OT Lüneburg Melchior und Fittkau GmbH, 21335 Lüneburg (DE)
(72) Erfinder: Melchior, Harald, 21379 Echem (DE)
(74) Vertreter: Rauschenbach, Marion

(57) **Zusammenfassung**

Die Erfindung bezieht sich auf das Gebiet der Medizintechnik und betrifft eine Orthese, welche beispielsweise für Knie-, Knöchel-, Ellenbogen- oder Schultergelenke anwendbar ist.

Aufgabe der vorliegenden Erfindung ist es daher, eine Orthese anzugeben, welche universeller einsetzbar und an die Probleme des Patienten sowie ihren Trainingszustand anpassbar ist.

Die Aufgabe wird gelöst durch eine Orthese, die mindestens enthält
- ein Gehäuse,
- und zwei Schienenaufnahmen oder ein oder zwei die Funktion der Schienenaufnahmen übernehmende Bauelemente,
- und zwei Stützelemente, die an den Körperbereichen befestigbar sind,
- und mindestens einen Blattfederstapel, der eine voreingestellte Federspannung aufweist,
- und mindestens ein Bauteil, welches die Federspannung der Blattfederstapel durch Druckausübung mindestens teilweise aufhebt,
- und mindestens ein Anschlagelement, welches einen Kontakt zur Kraftübertragung mit einem Ende des mindestens einen Blattfederstapels realisiert,
- und weiterhin mindestens zwei Begrenzungselemente, die die teilkreisförmige Bewegung des Anschlagselementes bei Bewegung des anatomischen Drehpunktes begrenzen.

## Beschreibung

Die Erfindung bezieht sich auf das Gebiet der Medizintechnik und betrifft eine Orthese, welche beispielsweise für Knie-, Knöchel-, Ellenbogen- oder Schultergelenke als anatomische Drehpunkte anwendbar ist.

Orthesen sind an sich allgemein bekannt als ein medizinisches Hilfsmittel für die Stabilisierung, Entlastung, Ruhigstellung, Führung oder Korrektur von Gliedmaßen oder des Rumpfes von menschlichen oder tierischen Körpern. Orthesen werden auch als Orthesengelenke bezeichnet. Stützstrukturen in Form von Schalen oder Schienen werden für den jeweiligen Anwendungsfall speziell gefertigt und an den Gliedmaßen befestigt. Im Bereich von Gelenken sind die Stützstrukturen auch mit gelenkartigen Strukturen versehen, um die gewünschte Beweglichkeit realisieren zu können. Die gewünschte Beweglichkeit kann in Abhängigkeit von den medizinischen und/oder therapeutischen Erfordernissen richtungs- und/oder betragsmäßig eingeschränkt und auch in Abhängigkeit von dem Fortschritt der Therapie geändert werden. In jedem Fall ist es bei den gelenkartigen Strukturen erforderlich, dass Strukturen vorhanden sind, die Rückstellkräfte oder -momente realisieren, um die Stützstruktur mit den gelenkartigen Strukturen in die Ausgangsstellung zurückführen zu können.

Weiter unterschieden wird bei der Bewegungsrichtung zwischen plantar und dorsal. Plantar bezieht sich auf die Fußsohle und dorsal bedeutet "in Gegenrichtung" und bezieht sich im Zusammenhang mit dem Begriff plantar damit auf den Knöchel. Im Zusammenhang mit anderen Körperteilen kann der Begriff dorsal ebenfalls angewandt werden, bezieht sich damit immer zu einem "Bezugskörperteil" in Gegenrichtung. Universeller könnten die Begriffe Flexion und Extension verwendet werden, die Beugung und Streckung in der Orthopädie und Orthopädietechnik beschreiben.

Die DE 10 2010 014 334 A1 beschreibt ein Orthesengelenk mit zwei Funktionsmitteln, die zum Bilden eines federnden Dorsalanschlags und eines federnden Plantaranschlags vorhanden sind. Die Funktionsmittel sind als Tellerfederanordnung ausgebildet und in Kanälen angeordnet. Die Kanäle sind in einem Grundkörper mit einer Schienenaufnahme für ein Oberteil ausgebildet und wirken auf einen schwenkbar an dem Grundkörper angeordneten Fußbügel.

Aus der DE 102013011 382 A1 ist ein Orthesengelenk mit einem Oberkörper bekannt, der eine Aufnahme für eine proximale Komponente und eine Lagerstelle für eine schwenkbar an den Grundkörper angeordnete distale Komponente, sowie auf die distale Komponente einwirkende Anschläge oder Federelemente aufweist.

Von der Fa. Fior & Gentz ist eine Systemknöchelgelenk-Orthese bekannt (NEURO SWING), durch welche die dynamische Aufrichtung des Patienten aus einer gebeugten Stellung sowie die Verbesserung der Gang- und Standsicherheit durch Ausbalancieren des Körpers erreicht wird. Unterschiedliche starke Federeinheiten, die in das Systemknöchelgelenk eingebaut sind, geben eine maximale Bewegungsfreiheit in Plantarflexion und Dorsalextension vor. Diese kann durch eine Bewegungslimitierungsschraube verringert werden. Die hintere Federeinheit ermöglicht ein kontrolliertes, dynamisches Absenken des Fußes in loading response. Außerdem dient das Systemgelenk zur Bewegungsführung mit integrierter Fußheberfunktion.

Aus der Vielzahl an Lösungsmöglichkeiten für ein Orthesengelenk ist ersichtlich, dass die jeweiligen konstruktiven Ausgestaltungen lediglich immer nur für einen begrenzten Einsatzzweck abgestimmt und einsetzbar sind. Durch diese relative Individualisierung der technischen Ausgestaltungen des Orthesengelenkes werden ihre Herstellung und Anwendungszeiten und Anwendungsbereiche für eine Vielzahl von Patienten eingeschränkt und damit teuer und uneffektiv. Ebenso sind nachträgliche Anpassungen an spezielle Bedürfnisse von Patienten meist nicht möglich, was insbesondere für Veränderungen durch Therapiefortschritte des Patienten nachteilig ist.

Aufgabe der vorliegenden Erfindung ist es daher, eine Orthese anzugeben, welche universeller einsetzbar und in der Größe für Patienten im Kindesalter bis Erwachsenenalter und an die Probleme des Patienten sowie ihren Trainingszustand anpassbar ist.

Die Aufgabe wird durch die in den Ansprüchen angegebene Erfindung gelöst. Vorteilhafte Ausgestaltungen sind Gegenstand der Unteransprüche, wobei die Erfindung auch Kombinationen der einzelnen Ansprüche im Sinne einer Verknüpfung miteinschließt, solange sie sich nicht gegenseitig ausschließen.

Die erfindungsgemäße Orthese enthält mindestens
- ein Gehäuse, in dem mindestens teilweise die Elemente der Orthese angeordnet sind,
- und zwei Schienenaufnahmen oder ein oder zwei die Funktion der Schienenaufnahmen übernehmende Bauelemente, die jeweils parallel zu den zu einem anatomischen Drehpunkt führenden Knochen angeordnet sind,
- und zwei Stützelemente, die jeweils parallel zu den zu einem anatomischen Drehpunkt führenden Knochen angeordnet sind und mit den Schienenaufnahmen oder den Bauelementen form- und/oder kraftschlüssig verbunden sind und an den Körperbereichen, für die die Orthese ausgebildet ist, befestigbar sind,
- und mindestens einen Blattfederstapel, der an einer Schienenaufnahme oder am Bauelement am dem anatomischen Drehpunkt abgewandten Ende angeordnet ist und mit der Schienenaufnahme oder dem Bauelement form- und/oder kraftschlüssig verbunden ist, und eine voreingestellte Federspannung aufweist, die in einem Winkel von 80° bis 110° entgegen der Längsachse der Schienenaufnahme oder dem Bauelement wirkend angeordnet ist,
- und mindestens ein Bauteil, welches an der Schienenaufnahme oder dem Bauelement, die mit dem Blattfederstapel verbunden sind, angeordnet ist und die Federspannung des oder der Blattfederstapel in Richtung der Längsachse der Schienenaufnahme oder des Bauelementes durch Druckausübung mindestens teilweise aufhebt,
- und mindestens ein Anschlagelement, welches an der Schienenaufnahme oder am Bauelement ohne Blattfederstapel am dem anatomischen Drehpunkt zugewandten Ende angeordnet ist und mindestens einen Kontakt zur Kraftübertragung mit einem dem anatomischen Drehpunkt zugewandten Ende des mindestens einen Blattfederstapels realisiert,
- und weiterhin mindestens zwei Begrenzungselemente, die an der Schienenaufnahme oder am Bauelement und/oder am Stützelement und/oder am Gehäuse angeordnet sind, und die die teilkreisförmige Bewegung des Anschlagelementes bei Bewegung des anatomischen Drehpunktes begrenzen.

Vorteilhafterweise ist anstelle der Schienenaufnahmen jeweils ein die Funktion der Schienenaufnahmen übernehmendes Bauelement vorhanden.

Weiterhin vorteilhafterweise ist wenigstens eine der Schienenaufnahmen oder Bauelemente gleichzeitig das Stützelement und/oder das Gehäuse für die Orthese.

Auch vorteilhafterweise ist die erfindungsgemäße Orthese insbesondere für ein Knöchelgelenk oder auch für ein Knie-, Ellenbogen- oder Schultergelenk als anatomische Drehpunkte einsetzbar.

Ebenfalls vorteilhafterweise liegen die Schienenaufnahmen oder Bauelemente in Form von Metallstäben oder kraftlinienorientierten Kunststoff-Compound-Netzwerken vor.

Und auch vorteilhafterweise sind das oder die Stützelemente und/oder das Gehäuse in Form von kraftlinienorientierten Kunststoff-Compound-Netzwerken ausgebildet.

Vorteilhaft ist es auch, wenn an mindestens einer Schienenaufnahme oder einem Bauelement an dem vom anatomischer Drehpunkt abgewandten Ende zwei Blattfederstapel angeordnet sind, die mit der Schienenaufnahme oder dem Bauelement form- und/oder kraftschlüssig verbunden sind und deren voreingestellte Federspannungen jeweils in einem Winkel von 80° bis 110° entgegen der Längsachse der Schienenaufnahmen oder der Bauelemente wirkend angeordnet sind, und an der Schienenaufnahme oder dem Bauelement je ein Bauteil für jeden Blattfederstapel vorhanden ist, welches die Federspannung je eines Blattfederstapels in Richtung der Längsachse der Schienenaufnahme oder des Bauelementes durch Druckausübung mindestens teilweise aufhebt.

Ebenfalls vorteilhaft ist es, wenn der oder die Blattfederstapel eine voreingestellte Federspannung von 500 Nmm bis 3500 Nmm aufweisen.

Weiterhin vorteilhaft ist es, wenn im Falle von zwei Blattfederstapeln diese die gleiche voreingestellte Federspannung aufweisen.

Und auch vorteilhaft ist es, wenn im Falle von zwei Blattfederstapeln diese an ihrem befestigten Ende mit einem Abstand zueinander positioniert sind.

Von Vorteil ist es auch, wenn die voreingestellte Federspannung des oder der Blattfederstapel jeweils in einem Winkel von 85° bis 95° entgegen der Längsachse der Schienenaufnahmen oder der Bauelemente wirkend angeordnet ist.

Auch von Vorteil ist es, wenn der oder die Schienenaufnahmen oder Bauelemente mittels einer Schraubverbindung mit dem oder den Stützelementen und/oder dem Gehäuses verbunden sind.

Ebenfalls von Vorteil ist es, wenn das Bauteil, welches auf den Blattfederstapel eine Druckausübung realisiert, in Form eines Bügels oder eines Stabes oder ausgeführt ist.

Weiterhin von Vorteil ist es, wenn das Bauteil die Druckausübung auf den oder die Blattfederstapel an der Stelle der größten Durchbiegung der Blattfederstapel durch die Vorspannung realisiert.

Und auch von Vorteil ist es, wenn die Federspannung durch die Druckausübung des oder der Bauteile vollständig aufgehoben ist.

Vorteilhaft ist es auch, wenn das Anschlagelement in Form einer würfelförmigen oder quaderförmigen oder zylinderförmigen Verlängerung in der Mitte des dem anatomischer Drehpunkt zugewandten oberen Endes der Schienenaufnahme oder Bauelementes positioniert ist.

Ebenfalls vorteilhaft ist es, wenn der Kontakt zur Kraftübertragung zwischen dem Anschlagelement und dem dem anatomischer Drehpunkt zugewandten Ende eines Blattfederstapels über eine Kontaktfläche zwischen Anschlagelement und Blattfederstapel realisiert ist, die über den gesamten teilkreisförmigen Bewegungsbereich des Anschlagelementes zumindest teilweise realisiert ist.

Und auch vorteilhaft ist es, wenn an den Schienenaufnahmen oder Bauelementen oder an den Stützelementen und/oder am Gehäuse mindestens zwei Begrenzungselemente befestigt sind, die die teilkreisförmige Bewegung des Anschlagselementes bei Bewegung des anatomischer Drehpunktes begrenzen, wobei je ein Begrenzungselement an je einem Ende der teilkreisförmigen Bewegung des Anschlagelementes angeordnet ist.

Weiterhin vorteilhaft ist es, wenn der anatomische Drehpunkt auf der Längsachse der Schienenaufnahmen oder Bauelemente angeordnet ist.

Vorteilhafterweise sind die Schienenaufnahmen oder Bauelemente und/oder die Bauteile und/oder die Anschlagelemente und/oder die Begrenzungselemente aus metallischen Werkstoffen oder faserverstärkten Werkstoffen, wie Kunststoff-Compounds, aufgebaut.

Mit der erfindungsgemäßen Lösung wird erstmals eine Orthese angegeben, welche universeller einsetzbar und in der Größe für Patienten im Kindesalter bis Erwachsenenalter und an die Probleme des Patienten sowie ihren Trainingszustand anpassbar ist.

Erreicht wird dies durch eine Orthese, welche mindestens ein Gehäuse enthält, in dem mindestens teilweise die Elemente der Orthese angeordnet sind.

Dabei sind vorteilhafterweise die Elemente der Orthese, die die Gelenkfunktion erfüllen und die die Stützfunktion erfüllen innerhalb des Gehäuses angeordnet.

Das Gehäuse soll möglichst leicht und optisch ansprechend ausgeführt sein.

Weiter enthält die erfindungsgemäße Orthese zwei Schienenaufnahmen oder ein oder zwei die Funktion der Schienenaufnahmen übernehmende Bauelemente, die jeweils parallel zu einem anatomischen Drehpunkt führenden Knochen angeordnet sind. Vorteilhafterweise ist anstelle der Schienenaufnahmen jeweils ein die Funktion der Schienenaufnahmen übernehmendes Bauelement vorhanden.

Ebenso enthält die erfindungsgemäße Orthese zwei Stützelemente, die jeweils parallel zu einem anatomischen Drehpunkt führenden Knochen angeordnet sind und mit den Schienenaufnahmen oder den Bauelementen form- und/oder kraftschlüssig verbunden sind und an den entsprechenden Körperbereichen, für welche die Orthese ausgebildet ist, befestigbar sind.

Vorteilhafterweise ist wenigstens eine der Schienenaufnahmen oder Bauelemente gleichzeitig das Stützelement und/oder das Gehäuse für die Orthese.

Ebenfalls vorteilhafterweise liegen die Schienenaufnahmen oder Bauelemente in Form von Metallstäben oder kraftlinienorientierten Kunststoff-Compound-Netzwerken vor.

Und auch vorteilhafterweise sind das oder die Stützelemente und/oder das Gehäuse in Form von kraftlinienorientierten Kunststoff-Compound-Netzwerken ausgebildet.

Weiter ist erfindungsgemäß mindestens ein Blattfederstapel vorhanden, der an einer Schienenaufnahme oder Bauelement an dem dem anatomischen Drehpunkt abgewandten Ende angeordnet ist und mit der Schienenaufnahme oder dem Bauelement form- und/oder kraftschlüssig verbunden ist, und eine voreingestellte Federspannung aufweist, die in einem Winkel von 80° bis 110° entgegen der Längsachse der Schienenaufnahme oder dem Bauelement wirkend angeordnet ist.

Dabei wird für die Längsachse der Schienenaufnahme oder des Bauelementes ein Winkel von 0° oder 180° angenommen.

Vorteilhafterweise weist die Orthese an mindestens einer Schienenaufnahme oder einem Bauelement an dem vom anatomischer Drehpunkt abgewandten Ende zwei Blattfederstapel auf, die mit der Schienenaufnahme oder dem Bauelement form- und/oder kraftschlüssig verbunden sind und deren voreingestellte Federspannungen jeweils in einem Winkel von 80° bis 110° entgegen der Längsachse der Schienenaufnahmen oder des Bauelementes wirkend angeordnet sind.

Vorteilhafterweise weist der oder die Blattfederstapel eine voreingestellte Federspannung von 500 Nmm bis 3500 Nmm auf.

Im Falle der vorteilhaften Anordnung von zwei Blattfederstapeln weisen die zwei Blattfederstapel jeweils die gleiche voreingestellte Federspannung auf, wobei die Federspannung beider Blattfederstapel entgegen der Längsachse der Schienenaufnahme oder des Bauelementes angeordnet ist.

Weiter vorteilhaft ist die voreingestellte Federspannung des oder der Blattfederstapel jeweils in einem Winkel von 85° bis 95° entgegen der Längsachse der Schienenaufnahmen oder der Bauelemente wirkend angeordnet.

Im Falle des Vorhandenseins von zwei Blattfederstapeln sind diese an ihrem befestigten Ende mit einem Abstand zueinander positioniert.

Erfindungsgemäß ist weiter mindestens ein Bauteil vorhanden, welches an der Schienenaufnahme oder dem Bauelement, die mit dem Blattfederstapel verbunden sind, angeordnet ist und die Federspannung des oder der Blattfederstapel in Richtung der Längsachse der Schienenaufnahme oder des Bauelementes durch Druckausübung mindestens teilweise, vorteilhafterweise vollständig, aufhebt.

Vorteilhafterweise ist das Bauteil, welches auf den Blattfederstapel eine Druckausübung realisiert, in Form eines Bügels oder eines Stabes oder ausgeführt.

Ebenfalls vorteilhafterweise realisiert das Bauteil die Druckausübung auf den oder die Blattfederstapel an der Stelle der größten Durchbiegung der Blattfederstapel durch die Vorspannung.

Erfindungsgemäß ist an der Orthese mindestens ein Anschlagelement vorhanden, welches an der Schienenaufnahme oder Bauelement ohne Blattfederstapel am dem anatomischer Drehpunkt zugewandten Ende angeordnet, welches mindestens einen Kontakt zur Kraftübertragung mit einem dem anatomischer Drehpunkt zugewandten Ende des mindestens einen Blattfederstapels realisiert.

Kraft ist ein grundlegender Begriff in der Physik. In der klassischen Physik versteht man darunter eine Einwirkung auf einen Körper, die ihn beschleunigt, das heißt seine Geschwindigkeit vergrößert, verringert, deren Richtung ändert oder die ihn verformt (Wikipedia, Stichwort Kraft).

Vorteilhafterweise ist das mindestens eine Anschlagelement in Form einer würfelförmigen oder quaderförmigen oder zylinderförmigen Verlängerung in der Mitte des dem anatomischer Drehpunkt zugewandten oberen Endes der Schienenaufnahme oder des positioniert.

Vorteilhafterweise wird der Kontakt zur Kraftübertragung zwischen dem Anschlagelement und dem dem anatomischer Drehpunkt zugewandten Ende eines Blattfederstapels über eine Kontaktfläche zwischen Anschlagelement und Blattfederstapel realisiert.

Diese Kontaktfläche muss während der Funktion der Orthese immer mindestens teilweise realisiert sein, damit der Kontakt zwischen Anschlagelement und Blattfederstapel über den gesamten teilkreisförmigen Bewegungsbereich des Anschlagelementes zumindest teilweise realisiert ist.

Erfindungsgemäß weist die Orthese weiterhin mindestens zwei Begrenzungselemente auf, die an den Schienenaufnahmen oder an den Bauelementen und/oder an den Stützelementen und/oder am Gehäuse angeordnet sind. Die zwei Begrenzungselemente begrenzen die teilkreisförmige Bewegung des Anschlagselementes bei Bewegung des anatomischer Drehpunktes.

Vorteilhafterweise sind die mindestens zwei Begrenzungselemente an je einem Ende der teilkreisförmigen Bewegung des Anschlagelementes angeordnet.

Beispielsweise ist bei dem erfindungsgemäßen Orthese der anatomische Drehpunkt, wie beispielsweise das Knie-, Knöchel-, Ellenbogen- oder Schultergelenk, aber auch andere Knochengelenke, auf der Längsachse einer Schienenaufnahme oder Bauelementes angeordnet und vorteilhafterweise in entgegengesetzter Richtung weg vom Anschlagelement angeordnet.

Besonders vorteilhaft ist die erfindungsgemäße Orthese an Scharniergelenken einsetzbar.

Weiter können vorteilhafterweise die Schienenaufnahmen oder die Bauelemente und/oder die Bauteile und/oder die Anschlagelemente und/oder die Begrenzungselemente aus metallischen Werkstoffen oder faserverstärkten Werkstoffen, wie Kunststoff-Compounds, aufgebaut sein.

Alle Elemente der Orthese können einzeln oder auch teilweise oder vollständig gemeinsam hergestellt werden, beispielsweise mittels additiver Fertigungsverfahren, wie 3D-Druck, Tailored Fiber Placement (TFP)-Verfahren, Resin Transfer Molding (RTM)-Verfahren, Vacuum Assisted Resin Infusion (VARI)-Verfahren, Fused Deposition Modeling (FDM)-Verfahren, Fused Filament Fabrication (FFF)-Verfahren.

Nachfolgend wird die Erfindung an einem Ausführungsbeispiel näher erläutert.

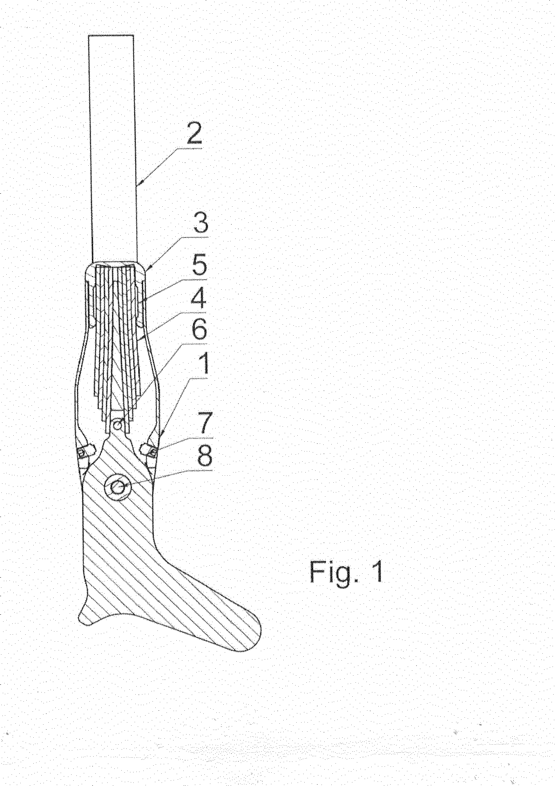

Dabei zeigt
- Fig. 1: die erfindungsgemäße Orthese in Ruheposition

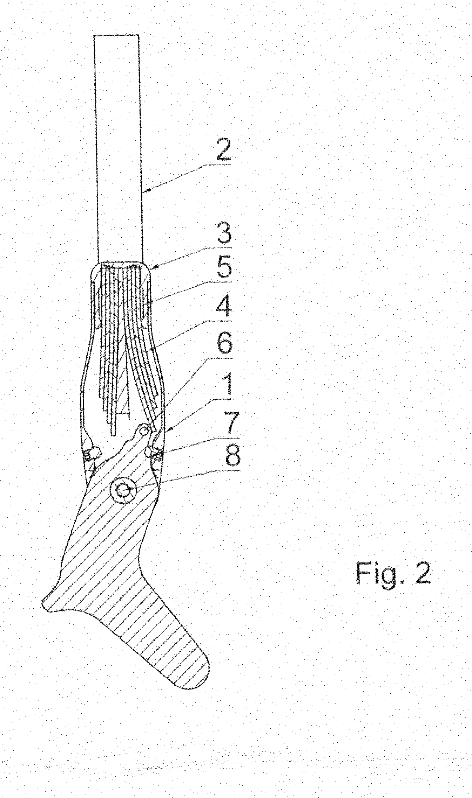

- Fig. 2: die erfindungsgemäße Orthese in einer Maximalpositionierung

### Beispiel 1

Für eine Fußgelenk-Orthese sind zwei Stützelemente 3 aus 3D-gedrucktem Titan vorhanden, eines für die Anpassung an die Wade und eines für die Anpassung an den Fuß.

Das Waden- Stützelement 3 beinhaltet eine längliche Schienenaufnahme 2. Die Schienenaufnahme am Wadenstützelement 3 besteht aus Stahl mit den Abmessungen 10 cm Länge, 1,6 cm Breite und 2 mm Dicke. Die Schienenaufnahmen 2 sind zu einem Drittel ihrer Länge im Titan der Stützelemente 3 mittels einer Schraubverbindung befestigt.

Am Waden-Stützelement 3 sind zwei Blattfederstapel 4 mittels einer Schraubverbindung befestigt, deren freie Enden in Richtung des anatomischen Drehpunktes 8, dem Fußgelenk, weisen. Die Blattfederstapel 4 sind mit einem Abstand von 2 mm zueinander befestigt.

Die Blattfederstapel 4 bestehen aus je vier Blattfedern mit den Abmessungen 60 mm Länge, 6 mm Breite des Blattfederstapels, 7 mm Dicke des Blattfederstapels und weisen eine Vorspannung von 1500 N/mm auf.

Die Vorspannung biegt die beiden Blattfederstapel 4 in der Mitte in einem Winkel von 90° von der Längsachse (0°) der Schienenaufnahme 2 weg.

Im Bereich der Befestigung der Blattfederstapel 4 am Waden-Stützelement 3 sind weiter zwei rundstabförmige Bauteile 5, jeweils mit den Abmessungen 10 mm lang und Durchmesser 5 mm vorhanden, die von der Befestigung bis zum oberen Drittel der Blattfederstapel 4 reichen, und welche den Blattfederstapel 4 in Richtung der Längsachse der Schienenaufnahme 2 durch Druckausübung drücken.

Durch die Wirkung der stabförmigen Bauteile 5 weisen die Blattfederstapel 4 an ihren freien Enden in Richtung des Fußgelenkes dann auch einen Abstand von 5,4 mm zueinander auf.

In der Mitte des freien Endes des Fuß-Stützelementes 3 ist ein zylinderförmiges Anschlagelement 6 vorhanden, welches mit der Herstellung es Fuß-Stützelementes 3 mittels 3D-Druck aus Titan gemeinsam hergestellt worden ist, und in Richtung des anatomischen Drehpunktes 8, des Fußgelenkes, weist.

Das Anschlagelement 6 hat die Abmessungen Breite 5,4 mm, Höhe 5 mm und steht im stofflichen Kontakt zur Kraftübertragung mit den Blattfederstapeln 4, das heißt, es reicht mit 4 mm in den Abstand der beiden Blattfederstapel 4 hinein.

Weiter sind an dem Waden-Stützelement 3 zwei Schrauben als Begrenzungselemente 7 mit 5 mm Durchmesser und 7 mm Länge eingeschraubt.

Durch die beiden Begrenzungselemente 7 wird die teilkreisförmige Bewegung des Anschlagselementes 6 bei Bewegung des anatomischen Drehpunktes 8, also des Fußgelenkes, begrenzt.

Die so ausgebildete Orthese wird durch die Stützelemente 3 an der Außenseite der Wade und dem Fuß positioniert, so dass die Drehachse der Orthese im Bereich des Fußteils, mit der Bewegungsachse des Fußgelenkes und Knöchels zusammenfällt. Durch die Höhe der Vorspannung der Blattfederstapel 4 ist das Moment einstellbar, das der Patient aufbringen muss, um die Schienenaufnahme 2 im Waden-Stützelement 3 gegenüber dem Anschlagelement 6 zu verdrehen und durch die Position und Größe der Anschlagelemente 6 in Ruheposition und im maximalen Drehwinkel ist der maximale Auslenkwinkel der Schienenaufnahme 2 am Waden-Stützelement 3 eingestellt.

So kann die Orthese für den Knöchel sowohl an den Therapiezustand der Patienten, als auch an deren Abmessungen und Alter angepasst werden.

### Beispiel 2

Für eine Ellenbogen-Orthese sind zwei Stützelemente 3 aus einem kraftlinienorientierten faserverstärkten Kunststoff-Netzwerk vorhanden, eines für die Anpassung an den Oberarm und eines für die Anpassung an den Unterarm.

Jedes der Stützelemente 3 beinhaltet eine längliche Schienenaufnahme 2 aus Aluminium mit den Abmessungen 10 cm Länge, 1,2 cm Breite und 10 mm Dicke. Die Schienenaufnahmen 2 sind zur Hälfte ihrer Länge im Kunststoff der Stützelemente 3 mittels einer Klebverbindung befestigt.

An dem freien Ende der Schienenaufnahme 2 am Oberarm und 2 mm von der Hälfte der Breite der Schienenaufnahme 2 beabstandet ist ein Blattfederstapel 4 mittels einer Klebverbindung befestigt, dessen freies Ende in Richtung des anatomischen Drehpunktes 8, des Ellenbogengelenkes, weist.

Der Blattfederstapel 4 besteht aus drei Blattfedern mit den Abmessungen 3 mm Dicke des Blattfederstapels, 7 mm Breite des Blattfederstapels und 60 mm Länge und weist eine Vorspannung von 800 N/mm auf.

Die Vorspannung biegt den Blattfederstapel 4 in der Mitte des Blattfederstapels in einem Winkel von 100° von der Längsachse der Schienenaufnahme 2 weg.

Im Bereich der Befestigung des Blattfederstapels 4 an der Schienenaufnahme 2 ist ein Bügel 5 mit den Abmessungen 5,4 mm Durchmesser und 20 mm Länge als Bauteil angeklebt, der bis zur Stelle der größten Durchbiegung des Blattfederstapels 4 reicht, und welcher den Blattfederstapel 4 in Richtung der Längsachse der Schienenaufnahme 2 durch Druckausübung drückt.

Durch die Wirkung des Bügels 5 ist der Blattfederstapel 4 im Abstand von 2 mm parallel zur Längsachse und in der Mitte der Schienenaufnahme 2 angeordnet.

In der Mitte des freien Endes der Schienenaufnahme 2 am Unterarm-Stützelement 3 ist ein zylinderförmiges Anschlagelement 6 durch eine Klebverbindung an der Schienenaufnahme 2 befestigt in Richtung des anatomischen Drehpunktes 8, des Ellenbogengelenkes.

Das Anschlagelement 6 hat die Abmessungen Breite 3 mm und Höhe 5 mm und steht im stofflichen Kontakt zur Kraftübertragung mit dem Blattfederstapel 4, das heißt, es reicht 10 mm über das freie Endes des Blattfederstapels 4 hinaus.

Weiter sind an dem Oberarm-Stützelement 3 zwei metallische Schrauben als Begrenzungselemente 7 mit den Abmessungen 4mm Durchmesser und 6 mm Länge vorhanden.

Durch die beiden Begrenzungselemente 7 wird die teilkreisförmige Bewegung des Anschlagselementes 6 bei Bewegung des anatomischen Drehpunktes 8, also des Ellenbogengelenkes, begrenzt.

Die so ausgebildete Orthese wird durch die Stützelemente 2 an der Außenseite der des Ober- und Unterarmes positioniert, so dass die Drehachse der Orthese im Bereich des Unterarmes mit der Bewegungsachse des Ellenbogengelenkes zusammenfällt. Durch die Höhe der Vorspannung des Blattfederstapels 4 ist das Moment einstellbar, das der Patient aufbringen muss, um die Schienenaufnahme 2 im Unterarm-Stützelement 3 gegenüber dem Blattfederstapel 4 zu verdrehen und durch die Position und Größe des Anschlagelementes 6 in Ruheposition und im maximalen Drehwinkel ist der maximale Auslenkwinkel der Schienenaufnahme 2 am Unterarm-Stützelement 3 eingestellt.

So kann die Orthese für den Ellenbogen sowohl an den Therapiezustand der Patienten, als auch an deren Abmessungen und Alter angepasst werden.

### Bezugszeichenliste

- 1: Gehäuse
- 2: Schienenaufnahme oder Bauelement
- 3: Stützelement
- 4: Blattfederstapel
- 5: Bauteil
- 6: Anschlagelement
- 7: Begrenzungselement
- 8: anatomischer Drehpunkt

## Patentansprüche

1. Orthese, enthaltend mindestens
- ein Gehäuse, in dem mindestens teilweise die Elemente der Orthese angeordnet sind,
- und zwei Schienenaufnahmen oder ein oder zwei die Funktion der Schienenaufnahmen übernehmende Bauelemente, die jeweils parallel zu den zu einem anatomischen Drehpunkt führenden Knochen angeordnet sind,
- und zwei Stützelemente, die jeweils parallel zu den zu einem anatomischen Drehpunkt führenden Knochen angeordnet sind und mit den Schienenaufnahmen oder den Bauelementen form- und/oder kraftschlüssig verbunden sind und an den Körperbereichen, für die die Orthese ausgebildet ist, befestigbar sind,
- und mindestens einen Blattfederstapel, der an einer Schienenaufnahme oder am Bauelement am dem anatomischen Drehpunkt abgewandten Ende angeordnet ist und mit der Schienenaufnahme oder dem Bauelement form- und/oder kraftschlüssig verbunden ist, und eine voreingestellte Federspannung aufweist, die in einem Winkel von 80° bis 110° entgegen der Längsachse der Schienenaufnahme oder dem Bauelement wirkend angeordnet ist,
- und mindestens ein Bauteil, welches an der Schienenaufnahme oder dem Bauelement, die mit dem Blattfederstapel verbunden sind, angeordnet ist und die Federspannung des oder der Blattfederstapel in Richtung der Längsachse der Schienenaufnahme oder des Bauelementes durch Druckausübung mindestens teilweise aufhebt,
- und mindestens ein Anschlagelement, welches an der Schienenaufnahme oder am Bauelement ohne Blattfederstapel am dem anatomischen Drehpunkt zugewandten Ende angeordnet ist und mindestens einen Kontakt zur Kraftübertragung mit einem dem anatomischen Drehpunkt zugewandten Ende des mindestens einen Blattfederstapels realisiert,
- und weiterhin mindestens zwei Begrenzungselemente, die an der Schienenaufnahme oder am Bauelement und/oder am Stützelement und/oder am Gehäuse angeordnet sind, und die die teilkreisförmige Bewegung des Anschlagelementes bei Bewegung des anatomischen Drehpunktes begrenzen.

2. Orthese nach Anspruch 1, bei der anstelle der Schienenaufnahmen jeweils ein die Funktion der Schienenaufnahmen übernehmendes Bauelement vorhanden ist, und/oder wenigstens eine der Schienenaufnahmen oder Bauelemente gleichzeitig das Stützelement und/oder das Gehäuse für die Orthese ist.

3. Orthese nach Anspruch 1, bei der die Orthese insbesondere für ein Knöchelgelenk oder auch für ein Knie-, Ellenbogen- oder Schultergelenk als anatomische Drehpunkte einsetzbar ist.

4. Orthese nach Anspruch 1, bei der die Schienenaufnahmen oder Bauelemente in Form von Metallstäben oder kraftlinienorientierten Kunststoff-Compound-Netzwerken vorliegen, und/oder bei der das oder die Stützelemente und/oder das Gehäuse in Form von kraftlinienorientierten Kunststoff-Compound-Netzwerken ausgebildet sind.

5. Orthese nach Anspruch 1, bei der an mindestens einer Schienenaufnahme oder einem Bauelement an dem vom anatomischer Drehpunkt abgewandten Ende zwei Blattfederstapel angeordnet sind, die mit der Schienenaufnahme oder dem Bauelement form- und/oder kraftschlüssig verbunden sind und deren voreingestellte Federspannungen jeweils in einem Winkel von 80° bis 110° entgegen der Längsachse der Schienenaufnahmen oder der Bauelemente wirkend angeordnet sind, und an der Schienenaufnahme oder dem Bauelement je ein Bauteil für jeden Blattfederstapel vorhanden ist, welches die Federspannung je eines Blattfederstapels in Richtung der Längsachse der Schienenaufnahme oder des Bauelementes durch Druckausübung mindestens teilweise aufhebt.

6. Orthese nach Anspruch 1, bei der der oder die Blattfederstapel eine voreingestellte Federspannung von 500 Nmm bis 3500 Nmm aufweisen.

7. Orthese nach Anspruch 1, bei der im Falle von zwei Blattfederstapeln diese die gleiche voreingestellte Federspannung aufweisen, und/oder bei der im Falle von zwei Blattfederstapeln diese an ihrem befestigten Ende mit einem Abstand zueinander positioniert sind.

8. Orthese nach Anspruch 1, bei der die voreingestellte Federspannung des oder der Blattfederstapel jeweils in einem Winkel von 85° bis 95° entgegen der Längsachse der Schienenaufnahmen oder der Bauelemente wirkend angeordnet ist.

9. Orthese nach Anspruch 1, bei der der oder die Schienenaufnahmen oder Bauelemente mittels einer Schraubverbindung mit dem oder den Stützelementen und/oder dem Gehäuses verbunden sind.

10. Orthese nach Anspruch 1, bei der das Bauteil, welches auf den Blattfederstapel eine Druckausübung realisiert, in Form eines Bügels oder eines Stabes oder ausgeführt ist, und/oder bei der das Bauteil die Druckausübung auf den oder die Blattfederstapel an der Stelle der größten Durchbiegung der Blattfederstapel durch die Vorspannung realisiert, wobei vorteilhafterweise die Federspannung durch die Druckausübung des oder der Bauteile vollständig aufgehoben ist.

11. Orthese nach Anspruch 1, bei der das Anschlagelement in Form einer würfelförmigen oder quaderförmigen oder zylinderförmigen Verlängerung in der Mitte des dem anatomischer Drehpunkt zugewandten oberen Endes der Schienenaufnahme oder Bauelementes positioniert ist.

12. Orthese nach Anspruch 1, bei der der Kontakt zur Kraftübertragung zwischen dem Anschlagelement und dem dem anatomischer Drehpunkt zugewandten Ende eines Blattfederstapels über eine Kontaktfläche zwischen Anschlagelement und Blattfederstapel realisiert ist, die über den gesamten teilkreisförmigen Bewegungsbereich des Anschlagelementes zumindest teilweise realisiert ist.

13. Orthese nach Anspruch 1, bei der an den Schienenaufnahmen oder Bauelementen oder an den Stützelementen und/oder am Gehäuse mindestens zwei Begrenzungselemente befestigt sind, die die teilkreisförmige Bewegung des Anschlagselementes bei Bewegung des anatomischer Drehpunktes begrenzen, wobei je ein Begrenzungselement an je einem Ende der teilkreisförmigen Bewegung des Anschlagelementes angeordnet ist.

14. Orthese nach Anspruch 1, bei der der anatomische Drehpunkt auf der Längsachse der Schienenaufnahmen oder Bauelemente angeordnet ist.

15. Orthese nach Anspruch 1, bei der die Schienenaufnahmen oder Bauelemente und/oder die Bauteile und/oder die Anschlagelemente und/oder die Begrenzungselemente aus metallischen Werkstoffen oder faserverstärkten Werkstoffen, wie Kunststoff-Compounds, aufgebaut sind.
